# EUROPÄISCHE PATENTSCHRIFT

(11) **EP 1 399 164 B1**
(45) Veröffentlichungstag und Bekanntmachung des Hinweises auf die Patenterteilung: **26.01.2005**
(21) Anmeldenummer: 02762280.2
(22) Anmeldetag: 04.06.2002
(51) Int. Cl.: A61K 31/555, A61K 31/28, A61K 31/30, A61P 31/10

(54) **METALLSALZE ALS ANTIMYKOTIKA**
METAL SALTS AS ANTIMYCOTIC AGENTS
SELS METALLIQUES COMME ANTIMYCOTIQUES

(30) Priorität: 05.06.2001 DE 10127244
(43) Veröffentlichungstag der Anmeldung: 24.03.2004
(73) Patentinhaber: BASF AKTIENGESELLSCHAFT, 67056 Ludwigshafen (DE)
(72) Erfinder: ZELLER, Dieter, 67346 Speyer (DE); TROPSCH, Jürgen, 67354 Römerberg (DE); SPEAKMAN, John-Bryan, 67273 Bobenheim (DE); NEUMANN, Peter, 68309 Mannheim (DE); HEIDENREICH, Hans-Peter, 76547 Sinzheim (DE)
(74) Vertreter: Pohl, Michael, Dr.
(86) Internationale Anmeldenummer: PCT/EP2002/006128
(87) Internationale Veröffentlichungsnummer: WO 2002/098430

(56) Entgegenhaltungen:
- EP-A- 0 803 192
- GB-A- 1 291 460
- US-A- 4 143 153

## Beschreibung

Die vorliegende Erfindung betrifft ein Metallsalz zur Verwendung in der Pharmazie, pharmazeutische Mittel, welche es enthalten und die Verwendung zur Herstellung eines pharmazeutischen Mittels zur Behandlung von Erkrankungen, die mit Mycobionten im Zusammenhang stehen.

Die Erfindung betrifft insbesondere die Verwendung eines Bis-(N-Organyldiazeniumdioxy)-Metallsalzes zur Herstellung eines Mittel zur Behandlung von Menschen und Tieren, die mit schädlichen Mikroorganismen, insbesondere Mycobionten befallen sind.

Es ist bekannt, dass Bis -(N-Organyldiazeniumdioxy)-Salze eine fungizide Wirkung haben. DT-OS 1 817 571 beschreibt beispielsweise eine Mischung aus Alkalihydroxid und einem Schwermetallsalz von N-Nitroso-N-cyclohexylhydroxylamin, die in Holzschutzmitteln als Fungizid eingesetzt wird. Ferner ist aus DE 24 10 603 ein Fungizid für den Holzschutz bekannt, welches Schwermetallsalzderivate von N-Nitroso-N-cyclohexylhydroxylamin enthält.

Obwohl zahlreiche antimycotische Mittel bekannt, wie beispielsweise Miconazol und Clotrimazol (Canesten®, Fa. Bayer), besteht ein ständiger Bedarf an neuen antimycotischen Wirkstoffen, die neue oder breitere Wirkungsspektren aufweisen oder gegenüber Pilzen aktiv sind, die Resistenzen gegenüber bekannten antimycotisehen Mitteln entwickelt haben.

Der vorliegenden Erfindung liegt daher die Aufgabe zugrunde, eine neue pharmazeutisch aktive, insbesondere antimycotisch wirksame, Verbindung zur Verwendung als Arzneimittel zur Verfügung zu stellen, die Nachteile der herkömmlichen Mittel behebt.

Es wurde nun überraschenderweise gefunden, dass verschiedene Bis-(N-Organyldiazeniumdioxy)-Metallsalze eine starke antimycotische Wirksamkeit gegenüber Organismen besitzen, die bei Menschen oder Tieren Mycosen verursachen können.

Die vorliegende Erfindung betrifft daher ein Metallsalz der Formel 1: 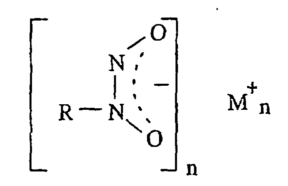 wobei
- R: für C₁-C₆-Alkyl, C₃-C₈-Cycloalkyl oder Aryl steht,
- M⁺: für ein Kationäquivalent steht, und
- n: für eine ganze Zahl von 1 bis 3 steht,
zur Verwendung als Arzneimittel.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl und 1-Ethyl-2-methylpropyl.

Bei der Cycloalkylgruppe handelt es sich vorzugsweise um eine C₅-C₇-Cycloalkylgruppe, wie Cyclopentyl, Cyclohexyl oder Cycloheptyl.

Bei der Arylgruppe handelt es sich vorzugsweise um Phenyl oder Tolyl.

M⁺ steht für ein Kationäquivalent, d. h. für ein einwertiges Kation, den einer positiven Einfachladung entsprechenden Anteil eines mehrwertigen Kations oder einer positiv geladenen metallatomhaltigen Gruppe. Beispielsweise steht M⁺ für ein Alkalimetallkation, wie z. B. Li⁺, Na⁺ oder K⁺. Geeignete zweiwertige Kationen sind z. B. Cu²⁺, Zn²⁺, Ni²⁺ und Co²⁺. Geeignete dreiwertige Kationen sind z. B. Fe³⁺ und Al³⁺. Geeignete einwertige metallatomhaltige Gruppen sind z. B. zinnhaltige Gruppen der Formel R^{a}R^{b}R^{c}Sn⁺, worin R^{a}, R^{b} und R^{c} unabhängig voneinander für C₁₋₆-Alkylreste stehen. Bevorzugt stehen R¹, R² und R³ für Butyl, d. h. die metallatomhaltige Gruppe steht für (C₄H₉)₃Sn⁺.

Bevorzugte Kationen sind K⁺, Cu²⁺ und Al³⁺. Besonders bevorzugt als Metall M ist Kupfer.

Bevorzugte Reste R sind C₅- und C₆-Alkyl oder C₅- und C₆-Cycloalkylgruppen, insbesondere Cyclohexyl.

Bevorzugte Metallsalze sind N-Cyclohexyldiazeniumdioxy-Kalium und Tris-(N-Cyclohexyldiazeniumdioxy)-Aluminium.

Eine besonders bevorzugte Ausführungsform betrifft die erfindungsgemäße Verwendung von Bis-(N-Cyclohexyldiazeniumdioxy)-Kupfer der Formel 2:

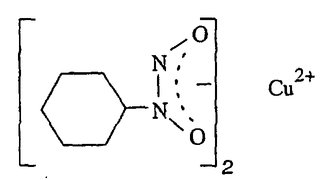

Ein weiterer Gegenstand der Erfindung ist ein pharmazeutisches Mittel, enthaltend wenigstens eine Verbindung der Formel 1, wie zuvor definiert, und wenigstens einen oder mehrere pharmazeutisch akzeptable(n). Träger- und/oder Zusatzstoff(e).

Die Erfindung betrifft auch Verfahren zur Behandlung von Erkrankungen, die mit Mycobionten im Zusammenhang stehen, wobei man einer Person oder einem Tier, die oder das einer derartigen Behandlung bedarf, eine antimycotisch wirkende Menge einer erfindungsgemäßen Verbindung der Formel 1 verabreicht.

Mycobionten, auch Pilze, Fungi oder Mycota genannt, sind eukaryotische Organismen, die unter aeroben Bedingungen wachsen und die benötigte Energie durch Oxidation organischer Substanzen gewinnen. Einige Vertreter, beispielsweise Hefen, sind fakultativ unter anaeroben Bedingungen lebensfähig, wobei die Energiegewinnung durch Gährungsprozesse erfolgt. Zu den Vertretern der Mycobionten zählen beispielsweise Hefe- oder Sprosspilze, Schimmelpilze, dimorphe Pilze und Dermatophyten.

Mycosen sind durch Pilze verursachte Erkrankungen, die lokal oder generalisiert auftreten können. Sie treten unter anderem bei Störungen des Immunsystems auf, z. B. bei Antibiotika- oder Cytostatikatherapien, bei der Verabreichung von Steroiden oder Hormonen, nach Bestrahlungen, bei parenteraler Ernährung, bei malignen Erkrankungen, Endokrinopathien oder Immundefekten. Bei systemischen Mycosen werden bevorzugt bestimmte Organe befallen. Dermatomycosen sind beispielsweise Erkrankungen, bei denen bestimmte Pilzarten, insbesondere Dermatophyten und Hefen die Haut und/oder deren Anhangsgebilde befallen. Dabei dringen die Pilze in Haut, Haare, Haarfolikel sowie Finger- oder Zehennägel ein und rufen Symptome wie Bläschenbildung, Exfoliation, Hautschrunde und Erosion hervor, meist verbunden mit Juckreiz und/oder allergischen Ekzemen. Während Dermatomycosen fast ausschließlich Haut, Haare und Nägel befallen, können sich durch Hefen verursachte Mycosen auch auf Schleimhäute und innere Organe ausdehnen.

Es wurde jetzt überraschenderweise gefunden, dass die Metallsalze der Formel 1, insbesondere die Kupfersalze davon, eine starke antimycotische Wirksamkeit aufweisen. Ein Beispiel einer besonders bevorzugten erfindungsgemäß verwendbaren Verbindung ist Bis-(N-Cyclohexyldiazeniumdioxy)-Kupfer. Das Wirkungsspektrum der erfindungsgemäß verwendbaren Verbindungen umfasst Hefen, Dermatophyten, Schimmelpilze, Pityrosporum ovale und biphasische Pilze. Die erfindungsgemäßen Verbindungen können daher erfolgreich als Wirksubstanz zur Behandlung zahlreicher lokaler und systemischer Mycosen bei Mensch und Tier eingesetzt werden. Besonders wirksam sind die erfindungsgemäßen Wirkstoffe zur Behandlung von Dermatomycosen, die durch Trichophyton rubrum, Trichophyton mentagrophyten und anderer Trichophytonarten, Microsporum canis, Epidermophyton floccosum sowie Scopulariopsis brevicaulis hervorgerufen werden, und Candidosen, die durch Candida tropicalis, Candida albicans, Candida glabrata, Candida parapsilosis und weitere Candidaarten verursacht werden.

Erkrankungen, bei denen die erfindungsgemäßen Verbindungen eingesetzt werden können, sind beispielsweise Störungen des Immunsystems, HIV-Infektionen, AIDS, Hauterkrankungen, Erkrankungen des Atmungswegs und Rachenraums, Systeminfektionen, lokale Infektionen, z. B. der Haut, Haare, oder Nägel, Schleimhautinfektionen, otitis, Pharyngitis, Pneumonie, Pyelonephritis, Cystitis, Endocarditis, Bronchitis und Arthritis.

Zur vorliegenden Erfindung gehören auch pharmazeutische Zubereitungen, die ein oder mehrere erfindungsgemäße Wirkstoffe und ein oder mehrere nicht-toxische, inerte pharmazeutisch geeignete Trägermaterialien und gegebenenfalls ein oder mehrere nicht-toxische, inerte pharmazeutisch geeignete Hilfsstoffe sowie ein oder mehrere nicht-toxische, inerte pharmazeutisch geeignete Zusatzstoffe enthalten.

Pharmazeutisch geeignete Materialien sind die im Bereich der Pharmazie, Lebensmitteltechnologie und angrenzenden Gebieten bekanntermaßen verwendbaren Stoffe, insbesondere die in einschlägigen Arzneibüchern gelisteten, deren Eigenschaften einer physiologischen Anwendung nicht entgegenstehen.

Die Herstellung der Bis-(N-Organyldiazeniumdioxy)-Metallsalze, die erfindungsgemäß als antimycotische Wirkstoffe eingesetzt werden, erfolgt nach herkömmlichen Verfahren, die dem Fachmann bekannt sind.

Die erfindungsgemäß verwendbaren Wirksubstanzen können beispielsweise als Tabletten, Dragees, Kapseln, Pillen, Granulate, Suppositorien, Lösungen, Suspensionen und Emulsionen, Pasten, Salben, Gele, Cremes, Lotionen, Puder oder Sprays formuliert sein.

Geeignete Trägermaterialien für Tabletten, Dragees, Kapseln, Pillen und Granulate sind übliche Füll- und Streckmittel, wie Stärken, Milchzucker, Rohrzucker, Glucose, Mannit und Kieselsäure.

Geeignete Trägermaterialien für Suppositorien, Lösungen, Suspensionen, Emulsionen, Pasten, Salben, Gele, Cremes und Lotionen sind ausgewählt unter Wasser, hydrophilen Komponenten und hydrophoben Komponenten sowie Mischungen davon. Geeignete hydrophile Trägerkomponenten sind z.B. ein-, zwei- oder mehrwertige Alkohole mit vorzugsweise 1 bis 8 Kohlenstoffatomen, wie Ethanol, n-Propanol, Isopropanol, Propylenglykol, Glycerin, Sorbit usw. Geeignete hydrophobe Trägerkomponenten sind z. B. Öl- oder Fettkomponenten, wie Paraffin und Paraffinöle; Vaselin; natürliche Fette und Öle wie Rizinusöl, Sojaöl, Maiskeimöl, Baumwollsaatöl, Erdnussöl, Olivenöl, Sonnenblumenöl, Sesamöl, Avocadoöl, Kakaobutter, Mandelöl, Pfirsichkernöl, Lebertran, Schweineschmalz, Walrat, Spermacetöl, Spermöl, Weizenkeimöl, Macadamianussöl, Nachtkerzenöl, Jojobaöl; Fettalkohole, wie Laurylalkohol, Myristylalkohol, Cetylalkohol, Stearylalkohol, Oleylalkohol; Fettsäuren wie Myristinsäure, Stearinsäure, Palmitinsäure, Ölsäure, Linolsäure, Linolensäure; Wachse wie Bienenwachs, Carnaubawachs, Candelillawachs, Walrat sowie Mischungen davon.

Geeignete Trägermaterialien für Puder oder Sprays sind beispielsweise Milchzucker, Talkum, Kieselsäure, Aluminiumhydroxid, Calciumsilikat und Polyamidpulver oder Gemische davon. Sprays können zusätzlich die üblichen Treibmittel, z. B. Fluorchlorkohlenwasserstoffe, enthalten.

Ferner können die erfindungsgemäßen Zubereitungen ein oder mehrere nicht-toxische, inerte pharmazeutisch geeignete Hilfsstoffe enthalten. Diese können feste, halbfeste oder flüssige Materialien sein, die als Vehikel, Träger oder Medium für den Wirkstoff dienen. Beispiele geeigneter Hilfsstoffe sind Gleitmittel, Netzmittel, emulgierende und suspendierende Mittel, konservierende Mittel, Adsorptionsmittel, Antioxidantien, Antireizstoffe, Bindemittel, Chelatbildner, Emulsionsstabilisatoren, Feuchthaltemittel, Filmbildner, Gelbildner, Geruchsmaskierungsmittel, Harze, Hydrokolloide, Lösemittel, Lösungsvermittler, Lösungsverzögerer, Neutralisierungsmittel, Permeationsbeschleuniger, Pigmente, quaternäre Ammoniumverbindungen, Resorptionsbeschleuniger, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle sowie weitere Verdünnungsmittel, Füllstoffe und Formulierungshilfsmittel jeder Art. Die Zumischung der Hilfsstoffe und/oder weiterer Zusatzstoffe, wie geruchs- und/ oder geschmacksverbessernder Zusätze oder Färbemittel erfolgt gewünschtenfalls in einer dem Fachmann bekannten Weise.

Die Tabletten, Dragees, Kapseln, Pillen oder Granulate können mit den üblichen Überzügen und Hüllen versehen sein, die gegebenenfalls Opakisierungsmittel enthalten. Sie können auch in microverkapselter Form vorliegen oder so zusammengesetzt sein, dass sie den oder die Wirkstoffe nur oder bevorzugt in einem bestimmten Teil des Intestinaltrakts, gegebenenfalls verzögert abgeben. Dabei können als Einbettungsmasse z. B. Polymersubstanzen und Wachse verwendet werden.

Zur parenteralen Applikation können erfindungsgemäß verwendbare Lösungen oder Emulsionen in steriler und blutisotonischer Form vorliegen.

Die erfindungsgemäßen pharmazeutischen Zubereitungen können in verschiedenen Dosierungseinheiten formuliert werden. Die Dosierungseinheiten können beispielsweise einer Einzeldosis, einem Bruchteil einer Einzeldosis oder einem Vielfachen davon entsprechen. Beispiele von Dosierungseinheiten sind ein, zwei, drei oder vier Einzeldosen oder eine halbe, drittel oder viertel Einzeldosis. Eine Einzeldosis enthält dabei vorzugsweise eine Wirkstoffmenge, die einer Tagesdosis entspricht oder der Hälfte, einem Drittel oder einem Viertel davon.

Die therapeutisch wirksamen Verbindungen in den oben genannten pharmazeutischen Zubereitungen sollten in einer Konzentration von etwa 0,0001 bis 99,5 Gew.-%, bevorzugt 0,001 bis 95 Gew.-%, speziell 0,01 bis 50 Gew.-%, bezogen auf die Gesamtmischung, enthalten sein. vorteilhafterweise wird bereits bei sehr geringen Wirkstoffkonzentrationen, wie 0,00015 Gew.-%, bei verschiedenen Mycosen eine therapeutische Wirksamkeit nachgewiesen. Die Zubereitungen können neben den erfindungsgemäßen Wirkstoffen auch weitere pharmazeutische Wirkstoffe enthalten.

Die Herstellung der erfindungsgemäßen pharmazeutischen Zubereitungen erfolgt in üblicher Weise nach dem Fachmann bekannten Verfahren.

Zur vorliegenden Erfindung gehört auch ein Verfahren zur Behandlung von Erkrankungen, die mit Mycobionten im Zusammenhang stehen. Dabei wird einer Person oder einem Tier, die oder das einer derartigen Behandlung bedarf, eine antimycotisch wirkende Menge des erfindungsgemäßen Wirkstoffs verabreicht. Der Wirkstoff oder die pharmazeutische Zubereitung kann lokal, oral, parenteral, intraperitoneal und/oder rectal verabreicht werden, vorzugsweise oral oder lokal.

Im Allgemeinen hat es sich als vorteilhaft erwiesen, den oder die erfindungsgemäßen Wirkstoffe bei der systemischen Verabreichung in einer Gesamtmenge von etwa 0,3 bis 80 mg/kg Körpergewicht, vorzugsweise 3 bis 15 mg/kg Körpergewicht je 24 Stunden zu verabreichen. Die Wirkstoffmenge kann auf einmal verabreicht werden oder auf mehrere Einzelgaben verteilt sein. In einigen Fällen kann es jedoch erforderlich sein, von den genannten Dosierungsvorschlägen abzuweichen, und zwar in Abhängigkeit vom Körpergewicht, der Art und Schwere der Erkrankung, der Art der Zubereitung oder der Verabreichungsform. So kann es in manchen Fällen ausreichen, eine geringere Wirkstoffmenge einzusetzen, während die oben genannte Wirkstoffmenge in anderen Fällen überschritten werden kann. Die jeweils am besten geeignetste Menge kann der Fachmann leicht ermitteln.

Gegenstand der Erfindung ist auch ein Mittel in Form einer Handelspackung mit wenigstens einem Mittel auf Basis eines Metallsalzes, wie zuvor definiert, zusammen mit Instruktionen für die therapeutische verwendung.

Gegenstand der Erfindung ist auch die Verwendung eines Metallsalzes der Formel 1, wobei R für C₁-C₆-Alkyl, C₃-C₈-Cycloalkyl oder Aryl steht, M⁺ für ein Kationäquivalent steht, und n für eine ganze Zahl von 1 bis 3 steht, zur Herstellung eines pharmazeutisehen Mittels zur Behandlung von Erkrankungen, die mit Mycobionten im Zusammenhang stehen.

Wird der erfindungsgemäße Wirkstoff als Futtermittelzusatz verwendet, kann er in üblicher Weise zusammen mit dem Futter bzw. der Futterzubereitung oder dem Trinkwasser gegeben werden.

Die Wirksamkeit des erfindungsgemäßen Wirkstoffs als antimycotisches Mittel wurde in einem in vitro-Agareinarbeitungstest untersucht. Dazu wurden verschiedene Mycobiontenproben in einem Nährmedium aus Sabouraud-Agar kultiviert und mit verschiedenen Wirkstoffmengen versetzt. Die Wirkstoffkonzentration im Medium lag im Bereich von 1 bis 100 ppm. Die Inkubationszeiten lagen zwischen 1 und 21 Tagen. Die Versuche mit Hefen des Candida-Typs zeigten eine wirksame Hemmung des Wachstums bei einer Wirkstoffkonzentration von 50 ppm und einer Inkubationszeit von 2 bis 5 Tagen. Versuche mit Dermatophytenkulturen zeigten eine wirksame Wachstumshemmung bei einer Wirkstoffkonzentration im Bereich von 15 bis 40 ppm nach einer Inkubationszeit von 1 bis 3 Wochen.

### Beispiele

### Beispiel 1:

Verwendung von Bis-(N-Cyclohexyldiazeniumdioxy)-Kupfer zur Wachstumshemmung verschiedener Candida-Arten

In einem Agareinarbeitungstest wurde die Wirksamkeit der erfindungsgemäßen Verbindung als Mittel zur Hemmung des Wachstums verschiedener Hefetypen untersucht. Dabei wurde als Nährmedium Sabouraud-Agar verwendet. Es wurden jeweils mehrere Proben nachstehender Hefetypen getestet, welche direkt von erkrankten Patienten isoliert wurden:
a) Candida tropicalis
b) Candida albicans
c) Candida glabrata
d) Candida parapsilosis

Die Inokulum-Suspensionen hatten eine Dichte von 10⁷ Kolonien bildende Einheiten/ml. Den Kulturen wurde Bis-(N-Cylohexyldiazeniumdioxy)-Kupfer in einer Menge zugesetzt, so dass eine Endkonzentration von 25 bzw. 50 ppm erreicht wurde. Anschließend wurden die Kulturen bei 30°C inkubiert und das Wachstum der Isolate nach einer Inkubationszeit von 2 bzw. 5 Tagen bewertet.

Bei einer Wirkstoffkonzentration von 25 ppm wurde bei allen getesteten Isolaten nach 2 und 5 Tagen Inkubationszeit ein fortschreitendes Wachstum beobachtet. Bei einer Wirkstoffkonzentration von 50 ppm hingegen blieb ein Wachstum aus.

### Beispiel 2:

### Verwendung von Bis-(N-Cyclohexyldiazeniumdioxy)-Kupfer zur Wachstumshemmung verschiedener Dermatophyten-Arten

In einem Agareinarbeitungstest wie in Beispiel 1 beschrieben wurde die Wirksamkeit der erfindungsgemäßen Verbindung zur Hemmung des Wachstums nachstehender Dermatophyten-Kulturen untersucht. Die Wirkstoffkonzentration in den Proben betrugen 2,5, 5, 10, 15, 20, 25, 30, 35, 40, 45 und 50 ppm. Die Isolate wurde nach einer Inkubationszeit von 7, 14 und 21 Tagen untersucht und das Wachstum ermittelt. Die Wirkstoffkonzentrationen, bei denen eine wirksame Hemmung des Wachstums beobachtet wurde, sind in nachstehender Tabelle gezeigt.

| Dermatophyt | Wirksame Wirkstoffkonzentration [ppm] | | |
|---|---|---|---|
| Inkubationsdauer | 7 Tage | 14 Tage | 21 Tage |
| Trichophyton rubrum | 15 | 15 | 15 |
| Trichophyton mentagrophytes | 30 | 30 | 30 |
| Microsporum canis | ≤ 10 | 15 | 15 |
| Epidermophyton floccosum | 15 | 20 | 20 |
| Scopulariopsis brevicaulis | 25 | 35 | 40 |

Die wirksamste Hemmung des Dermatophytenwachstums wurde nach einer Inkubationsdauer von 21 Tagen bei einer Konzentration von 15 bis 40 ppm beobachtet.

## Patentansprüche

1. Metallsalz der Formel 1 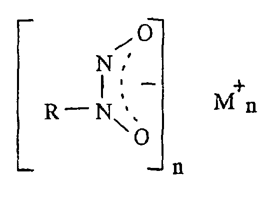 wobei
R für C₁-C₆-Alkyl, C₃-C₈-Cycloalkyl oder Aryl steht,
M⁺ für ein Kationäquivalent steht, und
n für eine ganze Zahl von 1 bis 3 steht,
zur Verwendung als Arzneimittel.

2. Metallsalz nach Anspruch 1, wobei M für ein zweiwertiges Metallkation aus der Gruppe aus Kupfer, Zink, Nickel und Cobalt steht.

3. Metallsalz nach Anspruch 2, wobei das Metallkation das Kupferkation ist.

4. Metallsalz nach einem der Ansprüche 1 bis 3, wobei es sich um Bis-(N-Cyclohexyldiazeniumdioxy)-Kupfer handelt.

5. Metallsalz nach einem der vorhergehenden Ansprüche zur Behandlung von Krankheiten, die mit Mycobionten im Zusammenhang stehen.

6. Pharmazeutisches Mittel, enthaltend wenigstens eine Verbindung der Formel 1, wie in Anspruch 1 definiert, und wenigstens einen oder mehrere pharmazeutisch akzeptable(n) Trägerund/oder Zusatzstoff(e).

7. Mittel in Form einer Handelspackung mit wenigstens einem Mittel auf Basis eines Metallsalzes, wie in einem der Ansprüche 1 bis 4 definiert, zusammen mit Instruktionen für die therapeutische Verwendung.

8. Verwendung eines Metallsalzes der Formel 1, wobei R für C₁-C₆-Alkyl, C₃-C₈-Cycloalkyl oder Aryl steht, M⁺ für ein Kationäquivalent steht, und n für eine ganze Zahl von 1 bis 3 steht, zur Herstellung eines pharmazeutischen Mittels zur Behandlung von Erkrankungen, die mit Mycobionten im Zusammenhang stehen.

## Claims

1. A metal salt of the formula 1 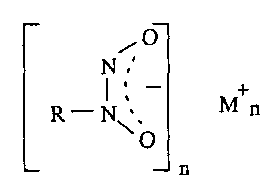 in which
R is C₁-C₆-alkyl, C₃-C₈-cycloalkyl or aryl,
M⁺ is a cation equivalent, and
n is an integer from 1 to 3,
for use as pharmaceutical.

2. A metal salt as claimed in claim 1 in which M is a bivalent metal cation selected from the group consisting of copper, zinc, nickel and cobalt.

3. A metal salt as claimed in claim 2, in which the metal cation is the copper cation.

4. A metal salt as claimed in any of claims 1 to 3, which is bis(N-cyclohexyldiazeniumdioxy)copper.

5. A metal salt as claimed in any of the preceding claims for treating diseases associated with mycobionts.

6. A pharmaceutical composition comprising at least one compound of the formula 1 as defined in claim 1 and at least one or more pharmaceutically acceptable carrier(s) and/or additive(s).

7. A composition in the form of a commercial pack with at least one composition based on a metal salt as defined in any of claims 1 to 4, together with instructions for therapeutic use.

8. The use of a metal salt of the formula 1 in which R is C₁-C₆-alkyl, C₃-C₈-cycloalkyl or aryl, M⁺ is a cation equivalent and n is an integer from 1 to 3 for the preparation of a pharmaceutical composition for treating diseases associated with mycobionts.

## Revendications

1. Sel métallique de formule 1 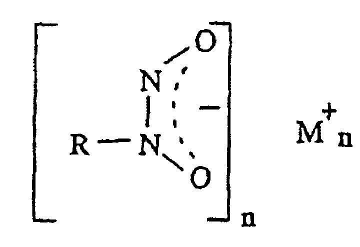 dans laquelle
R représente un groupe alkyle en C₁-C₆, cycloalkyle en C₃-C₈ ou aryle,
M⁺ représente un équivalent d'un cation, et
n représente un nombre entier allant de 1 à 3,
pour utilisation en tant que médicament.

2. Sel métallique selon la revendication 1, dans lequel M représente un cation métallique divalent choisi dans le groupe consistant en le cuivre, le zinc, le nickel et le cobalt.

3. Sel métallique selon la revendication 2, dans lequel le cation métallique est le cation cuivre.

4. Sel métallique selon l'une quelconque des revendications 1 à 3, qui est le bis(N-cyclohexyldiazéniumdioxy)-cuivre.

5. Sel métallique selon l'une quelconque des revendications précédentes, pour le traitement de maladies qui sont en relation avec des mycobiontes.

6. Produit pharmaceutique contenant au moins un composé de formule 1, telle que définie dans la revendication 1, et au moins un ou plusieurs véhicule(s) et/ou additif(s) pharmaceutiquement acceptable (s).

7. Produit sous forme d'un emballage du commerce, comportant au moins un produit à base d'un sel métallique tel que défini dans l'une quelconque des revendications 1 à 4, conjointement avec des directives pour l'utilisation thérapeutique.

8. Utilisation d'un sel métallique de formule 1, dans laquelle R représente un groupe alkyle en C₁-C₆, cycloalkyle en C₃-C₈ ou aryle, M⁺ représente un équivalent d'un cation, et n représente un nombre entier allant de 1 à 3, pour la fabrication d'un produit pharmaceutique destiné au traitement de maladies qui sont en relation avec des mycobiontes.
